# EUROPÄISCHE PATENTSCHRIFT

(11) **EP 0 873 727 B1**
(45) Veröffentlichungstag und Bekanntmachung des Hinweises auf die Patenterteilung: **22.09.2004**
(21) Anmeldenummer: 98106102.1
(22) Anmeldetag: 03.04.1998
(51) Int. Cl.: A61F 2/06

(54) **Radial aufweitbare Stützvorrichtung (Stent)**
Radial expandable support device (stent)
Dispositif de support expansible radialement (stent)

(30) Priorität: 25.04.1997 DE 19717475
(43) Veröffentlichungstag der Anmeldung: 28.10.1998
(73) Patentinhaber: Haindl, Hans, Dr., 30974 Wennigsen (DE)
(72) Erfinder: Haindl, Hans, Dr., 30974 Wennigsen (DE)
(74) Vertreter: Leine, Sigurd, Dipl.-Ing.

(56) Entgegenhaltungen:
- EP-A- 0 832 618
- DE-A- 19 537 872
- US-A- 5 697 971

## Beschreibung

Die Erfindung betrifft eine radial aufweitbare Stützstruktur der im Oberbegriff des Anspruchs 1 genannten Art zur Offenhaltung von Lumina innerhalb eines Körpers.

Durch EP 335 341 B1 ist eine Stützstruktur bekannt, die aus langgestreckten Gliederpaaren gebildet ist. Diese Stützstruktur wird in verengte Blutgefäße oder in andere, ein Lumen aufweisende Körperdurchgänge eingeschoben, um diese nach Erweiterung durch eine Ballondilatation offenzuhalten. Dabei wird die Stützstruktur in ihrem Durchmesser aufgeweitet, und sie verkürzt sich während der Aufweitung. Derartige Verkürzungen sind allerdings in der Regel unerwünscht, da diese Verkürzung dazu führt, daß eine wesentlich längere Stützstruktur in die Körperöffnung eingeführt werden muß, als sie am Einsatzort unmittelbar erforderlich ist. Diese so ausgebildete bekannte Stützstruktur paßt sich Bögen oder Kurven in den Körperöffnungen relativ schlecht oder gar nicht an, so daß zusätzliche Biegungselemente vorgesehen sind, wodurch die starren, rohrförmigen Abschnitte der Stützstruktur durch gelenkige Verbindungen biegbar miteinander verbunden sind. In der Praxis hat es sich gezeigt, daß in diesen gelenkigen Bereichen, bedingt durch die Dauerunruhe im Gewebelager, Gewebshypertrophien entsthehen können.

Durch US 5 697 971 ist eine radial aufweitbare Stützstruktur der im Oberbegriff des Anspruchs 1 genannten Art bekannt. Sie umfaßt einen rohrförmigen Körper mit einer sich zwischen einem ersten und einem zweiten Ende erstreckenden Wandfläche, die aus langgestreckten, miteinander verbundenen Gliedern gebildet ist. Diese Glieder bilden eine erste Gruppe von Gliedern, von denen benachbarte Glieder paarweise unter Bildung eines einen Zwischenraum umschließenden Gliederpaares an ihren Enden miteinander verbunden sind. Diese verbundenen Enden eines Gliederpaares sind mit den verbundenen Enden eines in Längsrichtung des rohrförmigen Körpers benachbarten Gliederpaares verbunden. Quer zur Längsausdehnung des rohrförmigen Körpers sind benachbarte Gliederpaare Paare von Gliedern entfernt von ihren miteinander verbundenen Enden unter Bildung eines die Längsachse des Rohrkörpers umlaufenden Ringes miteinander verbunden.

Der Erfindung liegt die Aufgabe zugrunde, eine radial aufweitbare Stützstruktur der im Oberbegriff des Anspruchs 1 genannten Art zur Offenhaltung von Lumina innerhalb eines Körpers, insbesondere eines Blutgefäßes, zu schaffen, das bei Aufweitung keine oder nur eine sehr geringe Längenverkürzung erfährt.

Die der Erfindung zugrundeliegende Aufgabe wird durch die im Kennzeichen des Anspruchs 1 angegebene Lehre gelöst.

Der Grundgedanke dieser Lehre besteht darin, die verbundenen Enden eines Paares von Gliedern mit den verbundenen Enden eines in Längsrichtung des rohrförmigen Körpers benachbarten Paares von Gliedern über langgestreckte Glieder einer zweiten Gruppe von Gliedern zu verbinden und die Schlitze von in Längsrichtung des rohrförmigen Körpers benachbarten Ringen zu überlappen, wobei die in Umfangsrichtung des rohrförmigen Körpers benachbarten Glieder mit einem Teil ihrer Länge neben jeweils zwei Schlitzen eines in Längsrichtung des rohrförmigen Körpers benachbarten Paares von Gliedern angeordnet sind. Die Paare von Gliedern benachbarter Ringe greifen also ineinander. Eine derartige Stützstruktur weist bei Ausdehnung nahezu keine Längenverkürzung auf, da die langgestreckten Glieder der zweiten Gruppe im Zusammenhang mit der Überlappung der benachbarten Ringe eine derartige Längenverkürzung ausgleichen. Bei der Ausweitung der Stützstruktur werden die ursprünglich in Längsrichtung des rohrförmige Körpers sich erstreckenden Glieder der ersten Gruppe an den Verbindungsstellen zwischen in Umfangsrichtung benachbarten Gliederpaaren verformt, beispielsweise geknickt, so daß sich die Schlitze aufweiten. Gleichzeitig wird die Überlappung geringer, so daß die Längenverkürzung ausgeglichen wird. Eine derartige Stützstruktur ist gleichzeitig in sich flexibel. Durch die Überlappung ist eine hohe Anzahl von Schlitzen in Längsrichtung gesehen möglich, so daß die einzelnen Glieder sehr dünn ausgeführt sein können, ohne daß die Wirkung der an die Aufweitung gekoppelten Längendehnung (durch Ausrichtung der langgestreckten Glieder der zweiten Gruppe aus der Längsrichtung heraus in die Umfangsrichtung), die zu der erwähnten Längenkompensation führt, verlorengeht.

Zweckmäßig ist es, daß jedes Ende eines Schlitzes mit den Enden der beiden durch die Überlappung benachbarten Schlitze eines benachbarten Ringes verbunden ist, um eine hohe Stabilität zu gewährleisten. Für eine hohe Flexibilität ist es vorteilhaft, daß jedes Ende eines Schlitzes mit dem Ende nur eines der beiden durch die Überlappung benachbarten Schlitze eines benachbarten Ringes verbunden ist. Dafür kann es auch vorteilhaft sein, daß nicht jeder Schlitz an seinen Enden mit einem Schlitz eines benachbarten Ringes verbunden ist.

In einer vorteilhaften Ausgestaltung können benachbarte Schlitze eines Ringes durch Stege miteinander verbunden sein. Die durch die Gliederpaare gebildeten Schlitze können die Form von Rauten haben, sie können als ovale oder Rechtecke ausgebildet sein. Die Schlitze können auch aus parallel verlaufenden langgestreckten Gliedern gebildet sein, wobei die Enden abgerundet miteinander verbunden sind. Durch derartige Ausbildungen kann das Biegeverhalten der Stützstrukturen beeinflußt werden. Desweiteren können die Glieder der ersten und zweiten Gruppe voneinander unterschiedliche Querschnitte aufweisen. Auch dadurch wird die Biegefähigkeit der Stützstruktur beeinflußt. Es ist auch möglich, daß der Querschnitt eines Gliedes sich über dessen Länge verändert, beispielsweise von der Mitte her zu den Enden hin verjüngt. Die Aufweitung erfolgt dann zunächst an den Enden der Gliederpaare; die entsprechende Materialverformung in dem mittleren Bereich der Schlitze, in dem diese mit benachbarten Schlitze eines Ringes verbunden sind, folgt dem nach. Dies hat zur Folge, daß alle Schlitze sich bei Krafteinwirkung gleichzeitig aufweiten. Es kann auch zweckmäßig sein, daß der Querschnitt der langgestreckten Glieder der ersten Gruppe an ihren Enden quadratisch ausgebildet ist.

Von Vorteil kann es auch sein, daß die Glieder der zweiten Gruppe von Gliedern die Enden der benachbarten Schlitze benachbarter Ringe nichtgeradlinig, also beispielsweise geschwungen miteinander verbinden. Zweckmäßig ist es weiterhin, mindestens vier, insbesondere sechs Schlitze in einem Ring um den Umfang des rohrförmigen Körpers herum benachbart zueinander anzuordnen. Als Material für die Stützstruktur kann vorzugsweise eines oder mehrere Metalle der Gruppe Tantal, Titan, Niob, Stahl, Platin oder eine Legierung mindestens eines dieser Metalle mit mindestens einem weiteren Metall (z.B. TaW, NbZr, TaNb, TiNb, TiAIV, Ptlr, jeweils mit geeigneten Gewichtsanteilen) verwendet werden. Dieses Material kann mit einem biokompatiblen Material beschichtet sein. Die rohrförmigen Körper sind aus nahtlosen Rohren gebildet, um Verspannungen zu vermeiden. Die Strukturen (die Anordnung der Glieder) sind durch Laserschweißen, Elektroerosion, Ätzen oder spanabhebend hergestellt.

Nachfolgend werden Ausführungsbeispiele der Erfindung anhand einer Zeichnung erläutert. In der Zeichnung zeigt:
- Figur 1: eine Stützstruktur, bei der jedes Ende eines Schlitzes mit jedem Ende eines benachbarten Schlitzes verbunden ist,
- Figur 2: die in Figur 1 gezeigte Stützstruktur in aufgeweitetem Zustand,
- Figur 3: eine Stützstruktur, bei der einige Schlitze an ihren Enden keine Glieder der zweiten Gruppe aufweisen,
- Figur 4: die Stützstruktur nach Figur 3, aufgeweitet und
- Figur 5: eine Stützstruktur nach Figur 3, mit geschwungenen Gliedern der zweiten Gruppe von Gliedern.

Die in den Figuren dargestellten Stützstrukturen weisen als Rauten ausgebildete Schlitze auf. Andersartig geformte Schlitze sind aus dem Stand der Technik hinreichend bekannt. Der Übersicht halber zeigen die Figuren einen Ausschnitt aus der Stützstruktur. Die aus Gliedern 1 einer ersten Gruppe von Gliedern gebildeten Gliederpaare, die jeweils einen Schlitz 2 bilden, sind in Längsrichtung der Stützstruktur ausgebildet und bilden zusammenhängend einen rohrförmigen Körper, wie er im Stand der Technik (zum Beispiel EP 221 570 B1 oder EP 335 341 B1) hinreichend beschrieben ist. Daher wird aus Gründen der Übersichtlichkeit in den Figuren lediglich ein Ausschnitt aus der aufgerollten Struktur dargestellt.

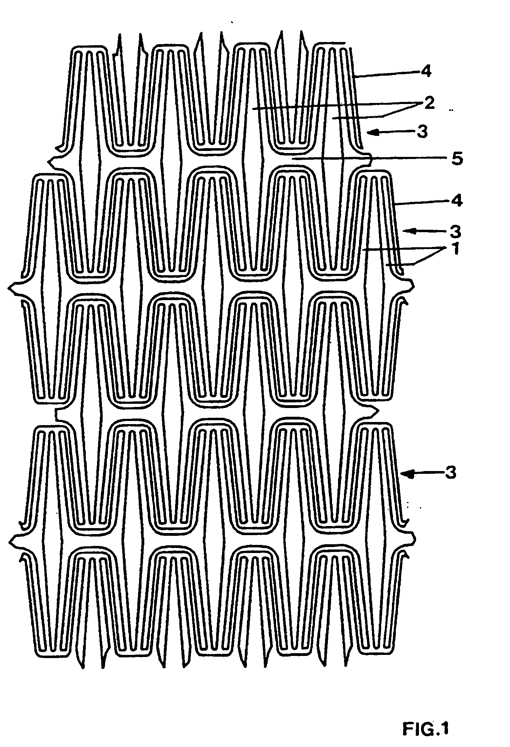

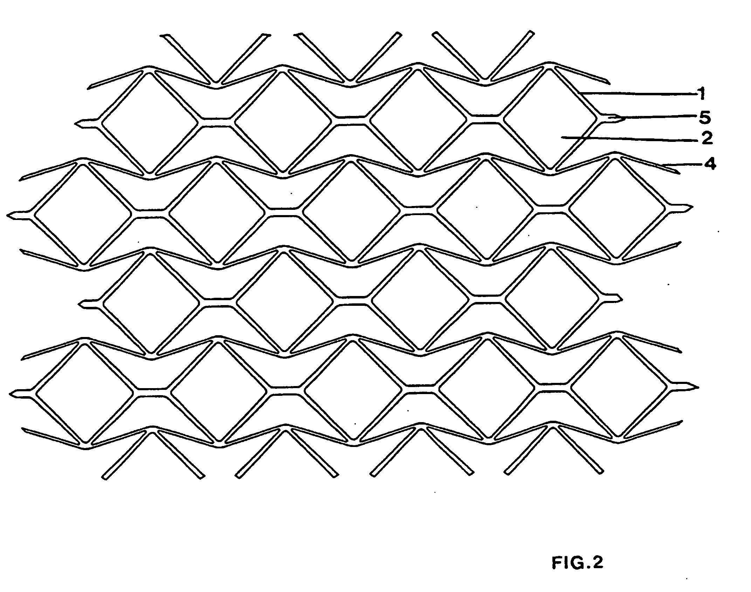

In der in Figur 1 dargestellten Stützstruktur sind die durch die Glieder 1 gebildeten Schlitze in Umfangsrichtung unter Bildung eines Ringes mit benachbarten Schlitzen 2 durch Stege 5 miteinander verbunden. In den durch die Stege 5 gebildeten Zwischenraum greift jeweils ein Ende eines Schlitzes 2 des benachbarten Ringes 3 ein. Die Enden der auf diese Weise benachbarten Schlitze 2 benachbarter Ringe 3 sind jeweils zu beiden Seiten mit Gliedem 4 einer zweiten Gruppe von Gliedern miteinander verbunden, so daß jeder Schlitz 2 an seinen Enden mit je zwei Schlitzen 2 benachbarter Ringe 3 verbunden ist. Diese Glieder 4 der zweiten Gruppe von Gliedern sind zunächst etwa parallel zu den Gliedern 1 der ersten Gruppe angeordnet. Bei Aufweitung der Stützstruktur richten sich diese in Umfangsrichtung der Stützstruktur aus, wobei die Enden der Schlitze 2 von den Stegen 5 benachbarter Ringe entfernt werden, so daß eine Längenkompensation der Stützstruktur erfolgt (Figur 2).

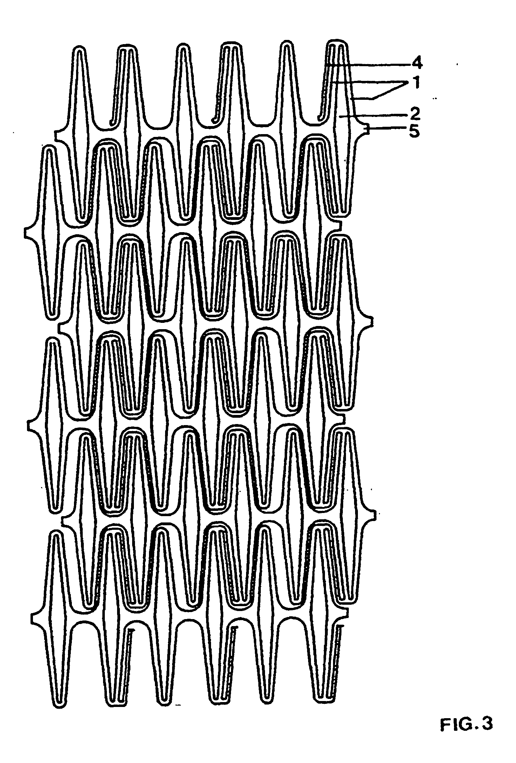

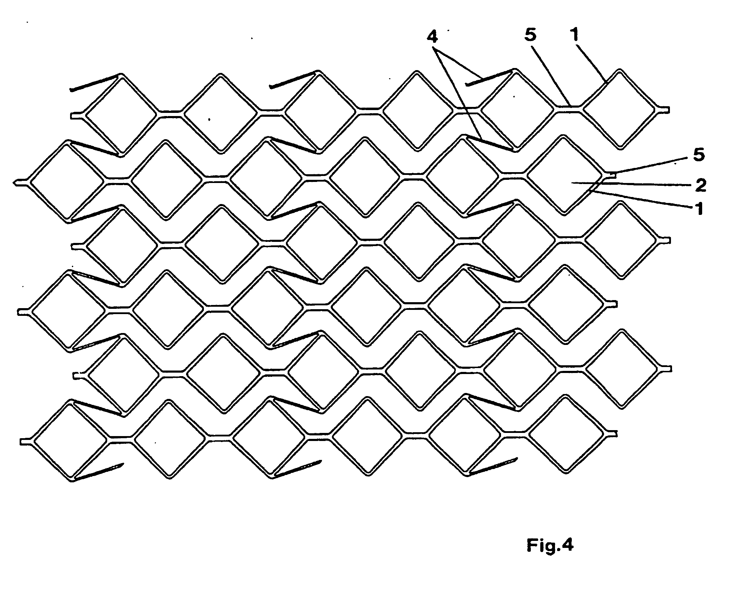

Eine ähnliche Stützstruktur wird in den Figuren 3 und 4 gezeigt. Im Unterschied zu der in Figuren 1 und 2 gezeigten Stützstruktur weist jeder zweite Schlitz 2 eines Ringes 3 an den Enden der Glieder 1 der ersten Gruppe keine Glieder 4 der zweiten Gruppe zur Verbindung mit den Enden benachbarter Schlitze auf. Eine solche Struktur ist flexibler als die in Figuren 1 und 2 gezeigte Stützstruktur.

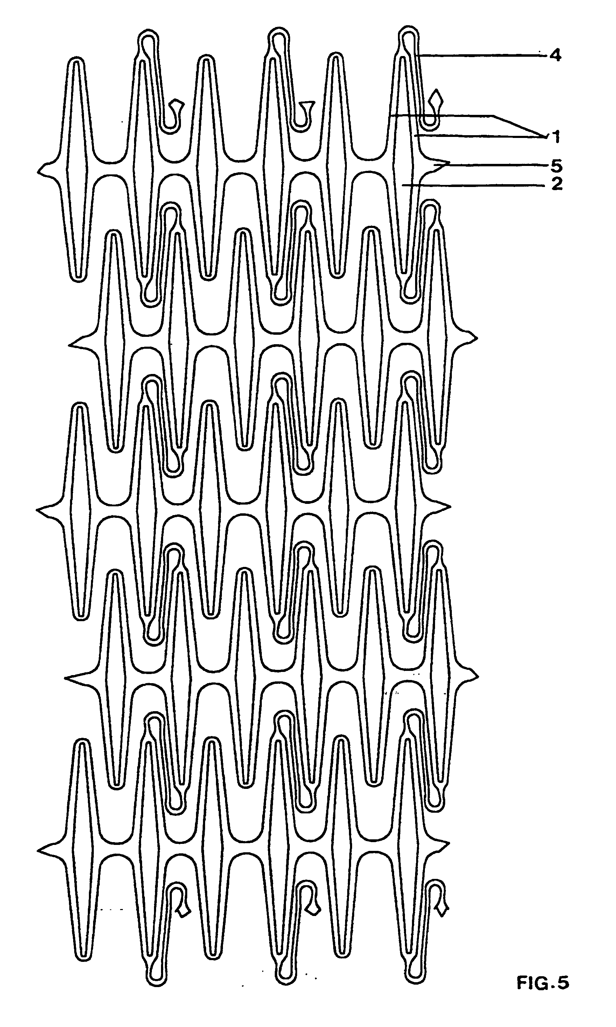

In Figur 5 ist eine Stützstruktur dargestellt, die der in Figur 3 gezeigten sehr ähnlich ist, wobei die Glieder 4 der zweiten Gruppe von Gliedern in diesem Beispiel mit einem Bogen an den Enden der Schlitze 2 ansetzen, so daß eine längere, als die geradlinige Verbindung zwischen Enden benachbarter Schlitze 2 sich überlappender Ringe 3 vorliegt, was eine Erhöhung der Flexibilität zur Folge hat. Auch hier sind, wie in den anderen Beispielen sechs Schlitze 2 auf einem Ring 3 angeordnet. Als Material wird in den gezeigten Beispielen ein medizinisch geeigneter Stahl verwendet. Dieser Stahl kann beschichtet sein mit einem biokompatiblen Material, wie es beispielsweise aus EP 335 341 B1 bekannt ist.

## Patentansprüche

1. Radial aufweitbare Stützstruktur zur Offenhaltung von Lumina innerhalb eines Körpers, insbesondere eines Blutgefäßes,
- die einen rohrförmigen Körper umfaßt mit einer sich zwischen einem ersten und einem zweiten Ende erstreckenden Wandfläche,
- die aus langgestreckten, miteinander verbundenen Gliedern gebildet ist,
- mit einer ersten Gruppe von Gliedern (1),
- wobei jeweils benachbarte Glieder (1) dieser ersten Gruppe paarweise unter Bildung eines einen Zwischenraum umschließenden Gliederpaares an ihren Enden miteinander verbunden sind und
- wobei quer zur Längsausdehnung des rohrförmigen Körpers benachbarte Paare von Gliedern entfernt von ihren miteinander verbundenen Enden unter Bildung eines die Längsachse des rohrförmigen Körpers umlaufenden Ringes (3) miteinander verbunden sind,
**dadurch gekennzeichnet,**
- **daß** die verbundenen Enden eines Paares von Gliedern (1) mit den verbundenen Enden eines in Längsrichtung des rohrförmigen Körpers benachbarten Paares von Gliedern (1) über langgestreckte Glieder (4) einer zweiten Gruppe von Gliedern (4) verbunden sind und
- **daß** jeder Schlitz (2) jeweils eines Ringes (3) von in Umfangsrichtung des rohrförmigen Körpers benachbarten Gliedern (1) mit einem Teil seiner Länge neben jeweils zwei Schlitzen (2) eines in Längsrichtung des rohrförmigen Körpers benachbarten Paares von Gliedern (1) angeordnet ist, derart, daß sich die Schlitze (2) von in Längsrichtung des rohrförmigen Körpers benachbarten Ringen (3) überlappen.

2. Radial aufweitbare Stützstruktur nach Anspruch 1, **dadurch gekennzeichnet, daß** die verbundenen Enden eines Paares von Gliedern (1) mit den verbundenen Enden eines in Längsrichtung des rohrförmigen Körpers benachbarten Paares von Gliedern (1) über jeweils ein langgestrecktes Glied (4) verbunden sind.

3. Radial aufweitbare Stützstruktur nach Anspruch 1, **dadurch gekennzeichnet, daß** die verbundenen Enden eines Paares von Gliedern (1) mit den verbundenen Enden eines in Längsrichtung des rohrförmigen Körpers benachbarten Paares von Gliedern (1) mit dem Ende nur eines der beiden benachbarten Schlitze (2) eines benachbarten Ringes (3) über ein langgestrecktes Glied (4) verbunden sind.

4. Radial aufweitbare Stützstruktur nach Anspruch 1, **dadurch gekennzeichnet, daß** nicht jedes verbundene Ende eines Paares von Gliedern (1) mit den verbundenen Enden eines in Längsrichtung des rohrförmigen Körpers benachbarten Paares von Gliedern (1) mit einem Schlitz (2) eines in Längsrichtung benachbarten Ringes (3) über ein langgestrecktes Glied (4) verbunden ist.

5. Radial aufweitbare Stützstruktur nach einem der Ansprüche 1 bis 4, **dadurch gekennzeichnet, daß** benachbarte Gliederpaare eines Ringe (3) durch Stege (5) miteinander verbunden sind.

6. Radial aufweitbare Stützstruktur nach einem der Ansprüche 1 bis 5, **dadurch gekennzeichnet, daß** die Schlitze (2) die Form von Rauten, Ovalen, Rechtecken aufweisen oder eine konstante Breite und abgerundete Verbindungsstellen an ihren Enden aufweisen.

7. Radial aufweitbare Stützstruktur nach einem der Ansprüche 1 bis 6, **dadurch gekennzeichnet, daß** die Glieder (1) der ersten Gruppe andere Querschnitte aufweisen als die Glieder (4) der zweiten Gruppe von Gliedern.

8. Radial aufweitbare Stützstruktur nach einem der Ansprüche 1 bis 7, **dadurch gekennzeichnet, daß** der Querschnitt der Glieder (1; 4) sich über die Länge der Glieder (1; 4) ändert.

9. Radial aufweitbare Stützstruktur nach einem der Ansprüche 1 bis 8, **dadurch gekennzeichnet, daß** die Glieder (4) der zweiten Gruppe von Gliedern als nichtgeradlinige Verbindung zwischen zwei Ringen (3) ausgebildet sind.

10. Radial aufweitbare Stützstruktur nach einem der Ansprüche 1 bis 9, **dadurch gekennzeichnet, daß** um den Umfang des rohrförmigen Körpers herum mindestens vier einen Schlitz (2) bildende Gliederpaare benachbart zueinander angeordnet sind.

11. Radial aufweitbare Stützstruktur nach einem der Ansprüche 1 bis 10, **dadurch gekennzeichnet, daß** sie im wesentlichen aus einem oder mehreren Metallen der Gruppe Tantal, Titan, Niob, Stahl, Platin oder eine Legierung mindestens eines dieer Metalle mit mindestens einem weiteren Metall gebildet ist.

12. Radial aufweitbare Stützstruktur nach einem der Ansprüche 1 bis 11, **dadurch gekennzeichnet, daß** sie mit einem biokompatiblen Material beschichtet ist.

## Claims

1. A radially expandable support structure for keeping open lumina within a body, in particular a blood vessel,
- which comprises a tubular body having a wall surface extending between a first and a second end,
- which is formed from elongated, interconnected members,
- having a first group of members (1),
- wherein respectively adjacent members (1) of this first group are connected in pairs to each other at their ends with the formation of a pair of members enclosing a space at their ends,
- wherein, at right angles to the longitudinal extension of the tubular body, adjacent pairs of members are connected to each other remote from their interconnected ends with the formation of a ring (3) around the longitudinal axis of the tubular body,
**characterised in that**
- the connected ends of a pair of members (1) are connected to the connected ends of a pair of members (1) adjacent in the longitudinal direction of the tubular body via elongate members (4) of a second group of members
- and **in that** each slit (2) of a respective ring (3) of members (1) adjacent in the circumferential direction of the tubular body is disposed with a part of its length next to respectively two slits (2) of a pair of members (1) adjacent in the longitudinal direction of the tubular body in such a manner that the slits (2) of rings (3) adjacent in the longitudinal direction of the tubular member overlap.

2. A radially expandable support structure according to Claim 1,
**characterised in that** the connected ends of a pair of members (1) are connected to the connected ends of a pair of members (1) adjacent in the longitudinal direction of the tubular body via an elongate member (4).

3. A radially expandable support structure according to Claim 1,
**characterised in that** the connected ends of a pair of members (1) with the connected ends of a pair of members (1) adjacent in the longitudinal direction of the tubular member are connected with the end of only one of the two adjacent slits (2) of an adjacent ring (3) via an elongate member (4).

4. A radially expandable support structure according to Claim 1,
**characterised in that** not every connected end of a pair of members (1) with the connected ends of a pair of members (1) adjacent in the longitudinal direction of the tubular body is connected to a slit (2) of an adjacent ring (3) in the longitudinal direction via an elongate member (4).

5. A radially expandable support structure according to one of Claims 1 to 4,
**characterised in that** adjacent pairs of members of a ring (3) are connected to each other by webs (5).

6. A radially expandable support structure according to one of Claims 1 to 5,
**characterised in that** the slits (2) have the form of lozenges, ovals, rectangles or have a constant width and rounded connection points at their ends.

7. A radially expandable support structure according to one of Claims 1 to 6,
**characterised in that** the members (1) of the first group have different cross sections than the members (4) of the second group of members.

8. A radially expandable support structure according to one of Claims 1 to 7,
**characterised in that** the cross section of the members (1; 4) changes over the length of the members (1; 4).

9. A radially expandable support structure according to one of Claims 1 to 8,
**characterised in that** the members (4) of the second group of members are constructed as a non-linear connection between two rings (3).

10. A radially expandable support structure according to one of Claims 1 to 9,
**characterised in that** at least four pairs of members forming a slit (2) are disposed adjacent to one another around the periphery of the tubular body.

11. A radially expandable support structure according to one of Claims 1 to 10,
**characterised in that** it is made substantially from one or more metals of the group comprising tantalum, titanium, niobium, steel, platinum or an alloy of at least one of these metals with at least one other metal.

12. A radially expandable support structure according to one of Claims 1 to 11,
**characterised in that** it is coated with a biocompatible material.

## Revendications

1. Structure de soutien expansible radialement pour maintenir ouverts des orifices à l'intérieur d'un corps, en particulier d'un vaisseau sanguin,
- qui comprend un corps de forme tubulaire avec une surface de paroi s'étendant entre une première et une deuxième extrémité,
- qui est formée par des éléments allongés, reliés entre eux,
- avec un premier groupe d'éléments (1),
- les éléments (1) voisins de ce premier groupe étant reliés entre eux deux par deux à leurs extrémités, pour former une paire d'éléments enfermant un espace intermédiaire,
- des paires d'éléments, voisines transversalement à l'étendue longitudinale du corps de forme tubulaire, étant reliées entre elles, à distance de leurs extrémités reliées entre elles, pour former un anneau (3) entourant l'axe longitudinal du corps de forme tubulaire,
**caractérisée en ce que**
les extrémités reliées d'une paire d'éléments (1) sont reliées aux extrémités reliées d'une paire d'éléments (1), voisine dans la direction longitudinale du corps de forme tubulaire, par des éléments (4) allongés d'un deuxième groupe d'éléments (4), et
**en ce que** chaque fente (2) d'un anneau (3) respectif d'éléments (1) voisins dans la direction périphérique du corps de forme tubulaire, est disposée par une partie de sa longueur, à côté de deux fentes (2) d'une paire d'éléments (1) voisine dans la direction longitudinale du corps de forme tubulaire, de manière que les fentes (2) de l'anneau (3) voisin dans la direction longitudinale du corps de forme tubulaire se chevauchent.

2. Structure de soutien expansible radialement selon la revendication 1, **caractérisée en ce que** les extrémités reliées d'une paire d'éléments (1) sont reliées aux extrémités reliées d'une paire d'éléments (1), voisine dans la direction longitudinale du corps de forme tubulaire, par un élément (4) allongé respectif.

3. Structure de soutien expansible radialement selon la revendication 1, **caractérisée en ce que** les extrémités reliées d'une paire d'éléments (1) sont reliées aux extrémités reliées d'une paire d'éléments (1), voisine dans la direction longitudinale du corps de forme tubulaire, par l'extrémité de l'une seulement des deux fentes (2) voisines d'un anneau (3) voisin, par l'intermédiaire d'un élément (4) allongé.

4. Structure de soutien expansible radialement selon la revendication 1, **caractérisée en ce que** toutes les extrémités reliées d'une paire d'éléments (1) ne sont pas reliées aux extrémités reliées d'une paire d'éléments (1), voisine dans la direction longitudinale du corps de forme tubulaire, par une fente (2) d'un anneau (3) voisin dans la direction longitudinale, par l'intermédiaire d'un élément (4) allongé.

5. Structure de soutien expansible radialement selon l'une des revendications 1 à 4, **caractérisée en ce que** des paires d'éléments voisines d'un anneau (3) sont reliées entre elles par des pontets (5).

6. Structure de soutien expansible radialement selon l'une des revendications 1 à 5, **caractérisée en ce que** les fentes (2) ont la forme de losanges, d'ovales, de rectangles ou présentent une largeur constante et des points de liaison arrondis à leurs extrémités.

7. Structure de soutien expansible radialement selon l'une des revendications 1 à 6, **caractérisée en ce que** les éléments (1) du premier groupe présentent des sections transversales différentes de celles des éléments (4) du deuxième groupe d'éléments.

8. Structure de soutien expansible radialement selon l'une des revendications 1 à 7, **caractérisée en ce que** la section transversale des éléments (1 ; 4) varie sur la longueur des éléments (1 ; 4).

9. Structure de soutien expansible radialement selon l'une des revendications 1 à 8, **caractérisée en ce que** les éléments (4) du deuxième groupe d'éléments sont réalisés en tant que liaison non rectiligne entre deux anneaux (3).

10. Structure de soutien expansible radialement selon l'une des revendications 1 à 9, **caractérisée en ce qu'**autour de la circonférence du corps de forme tubulaire sont disposées, voisines les unes des autres, au moins quatre paires d'éléments formant une fente (2).

11. Structure de soutien expansible radialement selon l'une des revendications 1 à 10, **caractérisée en ce qu'**elle est constituée pour l'essentiel en un ou plusieurs métaux du groupe tantale, titane, niobium, acier, platine ou en un alliage d'au moins l'un de ces métaux avec au moins un autre métal.

12. Structure de soutien expansible radialement selon l'une des revendications 1 à 11, **caractérisée en ce qu'**elle est revêtue d'un matériau biocompatible.
